# EUROPEAN PATENT APPLICATION

(11) **EP 4 162 973 A1**
(43) Date of publication of application: **12.04.2023**
(21) Application number: 22209524.2
(22) Date of filing: 18.10.2019
(51) Int. Cl.: A61M 60/13, A61M 60/216, A61M 60/865, A61M 25/06

(54) **SYSTEMS AND METHODS FOR MINIMIZING LEAKS DURING INSERTION OF PUMPS**

(30) Priority: 18.10.2018 US 201862747405 P
(62) Divisional of application: 19801153.8
(71) Applicant: Abiomed, Inc., Danvers, MA 01923 (US)
(72) Inventor: ZOLL, Jonathan, Danvers, 01923 (US)
(74) Representative: Klunker IP Patentanwälte PartG mbB

(57) **Abstract**

Systems and methods are described herein for preventing leaks across a hemostasis valve during insertion of an intracardiac pumping device. The intracardiac device placement system includes an intracardiac device, which comprises a pump with pump housing, a cannula, and a rotor. The intracardiac device placement system further includes an elongate catheter extending proximal of the pump housing, a sleeve configured to be removably disposed over a portion of the pump and covering at least one of the distal openings and the proximal openings, and an introducer comprising a tubular section with a hemostasis valve, the introducer configured to introduce the pump and the sleeve into a patient's vasculature while preventing the sleeve from wholly passing through the proximal hemostasis valve.

## Description

### Cross-Reference to Related Applications

This application claims the benefit of priority under 35 U.S.C. § 119(e) from United States Provisional Application Serial No. 62/747,405 filed October 18, 2018, the contents of which are hereby incorporated by reference in their entirety.

### Background

Intracardiac heart pump assemblies can be introduced into the heart either surgically or percutaneously and used to deliver blood from one location in the heart or circulatory system to another location in the heart or circulatory system. For example, when deployed in the heart, an intracardiac pump can pump blood from the left ventricle of the heart into the aorta, or pump blood from the right ventricle to the pulmonary artery. Intracardiac pumps can be powered by a motor located outside of the patient's body or a motor located inside the patient's body. Some intracardiac blood pump systems can operate in parallel with the native heart to supplement cardiac output and partially or fully unload components of the heart. Examples of such systems include the IMPELLA^{®} family of devices (Abiomed, Inc., Danvers MA).

An intracardiac device placement system includes a hemostasis valve and an introducer for introduction of an intracardiac device. An intracardiac device includes a pump, a cannula, and an elongate catheter coupled on its distal end to the pump. The intracardiac device is inserted into a patient's vasculature through the hemostasis valve and the introducer. Due to the blood pressure in the vasculature at the introducer site, when the intracardiac device is positioned across the hemostasis valve its hollow cannula allows blood to leak in a distal-to-proximal direction, with body fluids entering the intracardiac device through the cannula and exiting the intracardiac device through an opening on the other side of the hemostasis valve. Such leaks can be inconvenient for the physician placing the device, and/or dangerous for the patient. For example, leaks of pressurized fluid may result in the physician being sprayed, and/or a decrease in blood pressure for the patient.

### Summary

Systems, methods and devices are described herein for preventing leaks across a hemostasis valve during insertion of an intracardiac pumping device. The intracardiac device placement systems disclosed herein can prevent fluid leaks through the intracardiac device, for example through the cannula or a distal projection from the cannula. One element of such an intracardiac device placement system as described herein is a sleeve placed advantageously over a portion of the intracardiac device to seal at least one of the openings distal or proximal of the hemostasis valve, for example sealing the inlet and outlet area of the cannula. Advantageously, the sleeve closes off the leak paths present during insertion of the intracardiac device.

In some implementations, the intracardiac device comprises a pump, a cannula, a catheter proximal of the pump, and a distal projection that stabilizes the pump in a heart chamber or other vascular position. The placement system for the device includes a sleeve covering a portion of the cannula or pump and an introducer. For example, the blood pump system may be an Impella^{®} device of Abiomed, Inc. or any other intravascular blood pump. In some implementations, a controller is configured to facilitate operation of the blood pump systems described herein. For example, the controller may be the Automated Impella Controller (AIC)^{®} of Abiomed, Inc. or any other suitable controller that receives input signals and translates them into operational signals to operate the pump. At least one advantage of a separate controller configured to facilitate operation of the intracardiac blood pump systems is precise control of the system and the ability to acquire data related to the system.

In some implementations, the pump comprises a housing and a rotor disposed within the housing. The rotor may have at least one blade. Specifically, the rotor may include an impeller blade shaped to induce fluid flow when under rotational force. In some implementations, the rotor is driven by an implantable motor having a rotor and stator. A proximal end of the rotor may be coupled to a drive shaft. In some implementations, the motor is external to the patient and drives the rotor by an elongate mechanical transmission element, such as a flexible drive shaft, drive cable, or a fluidic coupling.

In some implementations, the catheter is an elongate multi-lumen catheter having a proximal end, a distal end and a central lumen. The distal end of the elongate multi-lumen catheter may be adjacent the pump housing. For example, when the blood pump system is in use, the pump housing is placed inside a patient's heart and the elongate multi-lumen catheter extends from the patient's heart and through the patient's vasculature such that a first portion of the catheter is within the patient and a second portion of the catheter is outside of the patient. The catheter may comprise two, three, four, five or any suitable number of lumens. For example, two separate tubes may pass through the central lumen of the catheter, thus defining three lumens total-the first central lumen, a lumen through the first tube and a lumen through the second tube. Some lumens may extend an entire length of the catheter, while other lumens may extend only partially through the catheter.

In some implementations, the catheter is catheter coupled on its distal end to the motor. In some implementations, the catheter is catheter coupled on its distal end to the pump housing. In some implementations, the catheter is coupled on its distal end to the pump housing and the pump further comprises a drive cable extending through the catheter.

In some implementations, the sleeve is configured to be removably disposed over a portion of the pump or cannula, covering at least one of the distal openings and the proximal openings of the pump or cannula. In some implementations, the cannula is covered by the sleeve and inserted with a distal portion of the sleeve through a hemostasis valve via an introducer into the patient's vasculature. In some implementations, proximal openings may be pump proximal openings and distal openings may be pump inlet openings, such that during device operation blood flows into the device through distal openings and exits the device through proximal openings. A flexible tip is positioned distal to the distal end of the inflow cage defining distal openings. The sleeve may cover the distal openings, proximal openings, or both. The distal openings may be positioned between the flexible tip and the distal end of the cannula, and the sleeve may cover such openings.

In some implementations, the sleeve is be configured to prevent leaks across a hemostasis valve. In some implementations, an inner diameter of the proximal end of the sleeve substantially matches an outer diameter of the elongate catheter such that, when disposed over the pump, the sleeve is fluidly sealed at its proximal end. The sleeve may form a lumen with a cross sectional diameter. The cross sectional diameter may taper from the distal end to the proximal end. For example, the cross sectional diameter may taper linearly from the distal end to the proximal end. As another example, the cross sectional diameter may start tapering at the middle of the sleeve to the proximal end. At least one advantage of tapering the sleeve is that it can be tapered to fit snugly around the elongate catheter, preventing fluid from leaking out of the sleeve.

In some implementations, the sleeve is configured to prevent fluid from flowing out of one of the distal openings or the proximal openings. In some implementations, when the sleeve covers the distal openings such that when the pump is located across the hemostasis valve, with the flexible atraumatic projection positioned distal of the hemostasis valve but the distal openings positioned proximal of the hemostasis valve, the sleeve prevents fluid from flowing from the vasculature of the patient through the lumen of the flexible atraumatic projection and out of the proximal openings. For example, the sleeve may cover the proximal or distal openings, or both, to prevent fluids from flowing in the distal to proximal direction. In some implementations, when the sleeve covers the proximal openings such that when the pump is located across the hemostasis valve, with the distal openings positioned distal of the hemostasis valve but the proximal openings positioned proximal of the hemostasis valve, the sleeve prevents fluid from flowing from the vasculature of the patient through the distal openings, and out of the proximal openings. For example, the sleeve may cover the proximal openings to prevent fluids from flowing in the distal to proximal direction. At least one advantage of preventing fluid from flowing out of one of the distal openings or the proximal openings (i.e., in the distal to proximal direction) located proximal of the hemostasis valve during insertion of the intracardiac device is to prevent body fluids such as blood from being sprayed towards the medical professional carrying out the insertion.

The sleeve may be made from any suitable material, including a thermoplastic. In some implementations, a portion of the sleeve is stiffer than the elongate catheter. In some implementations, a portion of the sleeve is stiffer than the cannula. At least one advantage of the stiffness of the portion of the sleeve being stiffer than the cannula is that the increased stiffness of the combination of the cannula and sleeve makes the insertion of the intracardiac device into the patient's vasculature easier. For example, during axillary insertions where the angle of insertion is greater a stiffer sleeve and cannula assembly may be easier to insert without buckling or kinking.

In some implementations, the sleeve may include a lip. The sleeve may include a lip at the proximal end of the sleeve. In some implementations, the sleeve includes a lip extending from an inner surface of a proximal end of the sleeve, the lip defining an inner diameter that approximates an outer diameter of the elongate catheter. For example, the inner diameter of the lip may be sized such that it forms a snug fit with the elongated catheter. In some implementations, the sleeve comprises a lip extending from an outer surface of the sleeve, the lip sized and shaped to abut against the hemostasis valve. When the intracardiac device is being inserted into the patient's vasculature, the sleeve may slide in a proximal to distal direction with the movement of the insertion of the intracardiac device. At a certain point in the sliding motion, the lip of the sleeve will come into contact with the proximal side of the hemostasis valve. The lip will prevent the proximal end of the sleeve going through the hemostasis valve. In some implementations the lip may be as a continuous body with the sleeve. The lip may be made of the same material as sleeve. The lip may be made from a material stiff enough to prevent deformations of the lip (i.e., to ensure that the lip does not bend, snap, or cause any other deformation that would allow the sleeve to be fully inserted into the introducer). At least one advantage of the lip is ensuring that the sleeve is never fully inserted into the introducer (i.e., and therefore is removable).

In some implementations, the intracardiac device placement system may include a handle. For example, the handle may be made of the same material as the sleeve. In some implementations the intracardiac device placement system comprises an ergonomic handle positioned over a portion of the sleeve. In some implementations the handle is integrally formed with the sleeve. At least one advantage of configuring the intracardiac device to include a handle is that the stiffness of the placement system is increased, including the sleeve and the cannula, enabling an easier entry of the intracardiac device into the patient's vasculature.

In some implementations the handle is formed of at least two pieces and is removable from the sleeve. For example, the two pieces of the handle may be configured to clip onto the sleeve (e.g., like a clamshell). For example, the two pieces may be configured to snap together around the sleeve. In some implementations at least two pieces of the handle are held in place around the sleeve by a removable pin. For example, the removal of the pin allows the two separate components to separate and be removed from the sleeve. In some implementations at least two pieces of the handle are held in place around the sleeve by a holding ring that screws into at least one of the at least two pieces of the handle. In some implementations, the handle may be configured as two separate pieces coupled around sleeve 800 that split apart when slid in a proximal to distal direction towards the proximal side of hemostasis valve 120. At least one advantage of a handle with two pieces are that the handle can be split on either side of the sleeve to remove the handle from the sleeve, allowing for more flexibility during the insertion procedure.

In some implementations, the intracardiac device placement system may include a locking mechanism, configured to prevent the sleeve from sliding freely over the pump or a portion of the pump. In some implementations the locking mechanism comprises a ring configured to rotate from a first, unlocked position to a second, locked position, wherein when in the second, locked position the ring clamps onto the sleeve, the elongate catheter, or both. In some implementations a handle positioned over a portion of the sleeve comprises the locking mechanism.

In some implementations, the locking mechanism includes a plurality of tabs, each tab of the plurality of tabs including a living hinge and a protrusion configured to fit at least partially within the distal openings, the proximal openings or both, such that the plurality of tabs snap into a locked position at the distal openings, the proximal openings or both. Tabs are configured inside the proximal end of the sleeve to form a locking mechanism, preventing the proximal end of the sleeve from passing through the hemostasis valve. The tabs may be made from a material stiff enough to prevent deformations of the tabs (i.e., to ensure that the tabs do not bend, snap, break, or cause any other deformation that would allow the sleeve to be fully inserted into introducer). In some implementations, the tabs are configured to be released from the locked position by applying a radially inward force to a distal portion of the sleeve. In some implementations, each tab of the plurality of tabs is sized and shaped to cover one of the distal openings in the locked position, and wherein each opening of the distal openings is covered by a tab of the plurality of tabs. In some implementations, each tab of the plurality of tabs is sized and shaped to cover one of the proximal openings in the locked position, and wherein each opening of the proximal openings is covered by a tab of the plurality of tabs. At least one advantage of including inner tabs to lock into distal openings, proximal openings, or both distal openings and proximal openings is that the locked position provides a stable and tight seal, such that fluid will not leak out between the openings and the tabs when relatively high fluid pressures and forces are applied during insertion.

In some implementations, the introducer comprising a tubular section with a hemostasis valve, the introducer configured to introduce the pump and the sleeve into patient vasculature while preventing the sleeve from wholly passing through the proximal hemostasis valve.

### Brief Description of the Drawings

FIG. 1 shows a placement system comprising an introducer configured to introduce an intracardiac device into a patient's vasculature, according to some implementations;
FIG. 2 shows a placement system comprising an introducer configured to introduce an intracardiac device into a patient's vasculature, according to some implementations;
FIG. 3 shows a placement system that includes a sleeve with a tapered inner diameter configured to fit snugly around an elongate catheter, according to certain implementations;
FIG. 4 shows a placement system that includes a sleeve with a lip extending radially outward from an outer surface of a sleeve, according to certain implementations;
FIG. 5 shows a placement system that includes a sleeve with an inner lip, according to certain implementations;
FIG. 6 shows a placement system that includes a sleeve with a locking mechanism extending radially outward from an outer surface of the sleeve, according to some implementations;
FIG. 7 shows a placement system that includes a sleeve with a locking mechanism and handle extending radially outward from an outer surface of the sleeve, according to some implementations;
FIG. 8 shows a placement system that includes a sleeve with a locking mechanism and handle extending radially outward from an outer surface of the sleeve, according to some implementations;
FIG. 9 shows a placement system that includes a sleeve with inner tabs extending radially inward from an inner surface of the sleeve, according to certain implementations; and
FIG. 10 shows a flowchart for preventing leaks across a hemostasis valve, according to certain implementations.

### Detailed Description

To provide an overall understanding of the systems, method and devices described herein, certain illustrative embodiments will be described. Although the embodiments and features described herein are specifically described for use in connection with a blood pump system, it will be understood that all the components and other features outlined below may be combined with one another in any suitable manner and may be adapted and applied to other types of cardiac therapy and cardiac assist devices, including cardiac assist devices implanted using a surgical incision and the like. Additionally, though the application of pump elements has been described here with regard to blood pumps, it is to be understood that the pump elements may be applied to other pumps for which any type of fluid flow being sent distally can flow proximally and damage electronic components. Although the embodiments and features described herein are specifically described for use in connection with an intracardiac blood pump system, it will be understood that a blood pump system according to the embodiments and features described herein may be used within any vasculature and/or in combination with other systems. For example, the sleeve systems and placement described below may be used in urethra or bladder catheterization systems; right heart cardiac support systems; intra-aortic balloon pumps; extracorporeal membrane oxygenation devices; left ventricular assist devices; renal support systems, such as cardiac assist devices to adjust kidney autoregulation; infusion systems; central venous catheters; or any other suitable system.

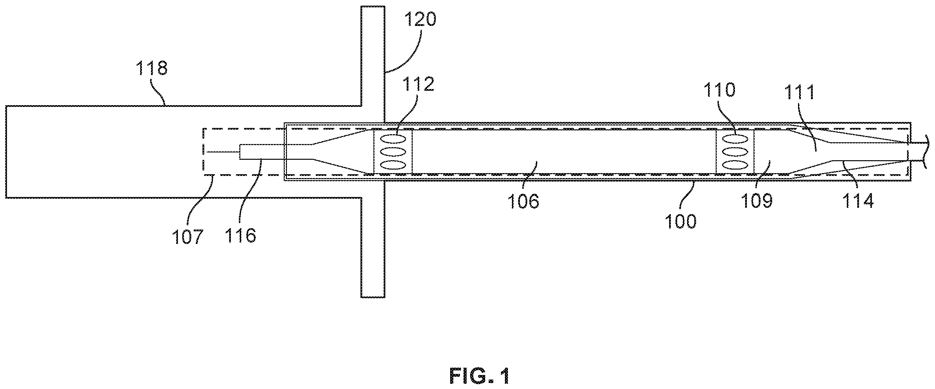

FIG. 1 shows a placement system comprising an introducer 118 configured to introduce an intracardiac device 122 into a patient's vasculature, according to some implementations. The placement system includes sleeve 100, introducer 118, and hemostasis valve 120. FIG. 1 shows an exemplary positioning of introducer 118 with intracardiac device 122 being inserted into introducer 118 - the distal end of intracardiac device 122 is already in the introducer 118 whereas the proximal end of intracardiac device 122 is not yet in the introducer. Introducer 118 is positioned such that the distal end of introducer 118 is located within the patient's vasculature, while the proximal end of introducer 118 is located outside the patient's body. Once introducer 118 is positioned within the patient's vasculature, an intracardiac device may be inserted into the proximal end of introducer 118 and fed through introducer 118 such that the device enters the patient's vasculature.

The intracardiac device comprises a pump 107 and a cannula 106. Pump 107 comprises a pump housing 109, a rotor (not shown), proximal openings 110, and distal openings 112, that are positioned proximal of the pump housing 109. The pump is configured to be operated by a motor within motor housing 111. Elongate catheter 114 is coupled on its distal end to the motor housing 111. Elongate catheter 114 defines a central lumen therein. In some implementations, elongate catheter 114 may be coupled to pump housing 109. The proximal end of cannula 106 interfaces with the distal end of the pump housing 109. The distal end of cannula 106 interfaces with a pump inflow cage that defines distal openings 112. Cannula 106 defines a lumen therein. In some implementations, proximal openings 110 may be pump proximal openings and distal openings 112 may be pump distal openings, such that during device operation blood flows into the device through distal openings 112 and exits the device through proximal openings 110. A flexible tip 116 is positioned distal to the distal end of the inflow cage defining distal openings 112. The intracardiac device defines at least one lumen therethrough - i.e., the central lumen of elongate catheter 114 may be in fluid communication with the interior lumen of cannula 106 during operation of the device (e.g., via a purge system such as that described below).

In some implementations, the motor is "onboard," as shown in FIG. 1, and may be located within the patient's body during operation of the pump and be configured with electrical leads that transmit power to the motor for driving the pump. The motor can alternatively be located outside of the patient's body and can actuate the rotor via a drive shaft, drive cable, or drive line. For example, the motor may be located within a handle of the intracardiac device. In some examples, a drive cable may extend through elongate catheter body 114 to a rotor located near a proximal end of cannula 106. In some implementations, the drive shaft, drive cable, or drive line operate in combination with a purge fluid delivery system.

Introducer 118 comprises a hemostasis valve 120. Hemostasis valve 120 is part of introducer 118 and is designed to have medical devices inserted through and be able to be removed in-line from the inserted medical devices. The intracardiac device is positioned across hemostasis valve 120 such that distal openings 112 are distal hemostasis valve 120 and proximal openings 110 are proximal hemostasis valve 120. This configuration may occur, for example, during insertion of the intracardiac device into the patient's vasculature as the device is passed through hemostasis valve 120.

In some implementations, purge fluid may flow through the pump to prevent ingress of blood cells into the pump. Alternatively or additionally, the purge fluid may function as a lubricant for bearings of the pump (not shown) or as a coolant to dissipate heat produced by electromagnetic motor coils of the motor stator. The purge fluid may be lubricant, coolant, medicine or any suitable hemocompatible fluid. For example, the purge fluid may be saline, Ringer's solution, glucose solution, heparin or any other suitable fluid. The purge fluid prevents blood from entering the motor housing 111 during operation of the pump 107. The purge fluid may also prevent ingress of blood into the elongate catheter body 114. In some implementations, a highly viscous purge fluid, such as a glucose solution, is used to lubricate bearings internal to the pump 107. In other implementations, pharmacological agents are used as a purge fluid to purge the pump of blood, as well as perform a medical purpose. For example, the purge fluid may include heparin to prevent blood clotting. The purge fluid flows through a lumen of the elongate catheter body 114 and flows out of the pump 107 at the proximal openings 110. The purge fluid is safely dispersed into the blood stream of the patient.

In some implementations, during insertion of the intracardiac device into the patient's vasculature as the device is passed through hemostasis valve 120, there are flow paths that allow fluids to flow in a distal-to-proximal direction. This is particularly true, for example, for auxiliary insertion of the intracardiac device where blood pressure is higher than in a femoral insertion.

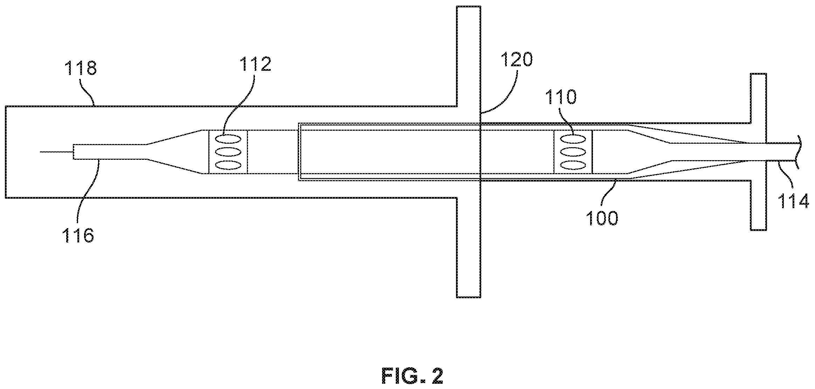

For example, there are two prominent leak paths during insertion. These leak pathways are created as the device is inserted through the introducer 118 in a proximal to distal direction. A first leak pathway occurs when, during insertion, flexible tip 116 is positioned distal hemostasis valve 120 and proximal openings 110 and distal openings 112 are both proximal hemostasis valve 120. The lumen through flexible tip 116 and to distal openings 112 can create a pathway for fluid to leak through hemostasis valve 120. The second leak pathway occurs, when, during insertion, distal openings 112 are distal of hemostasis valve 120 but proximal openings 110 are proximal hemostasis valve 120. Body fluids such as blood can flow through distal openings 112 through the cannula 106 and out proximal openings 110. The first path is shown/addressed in relation to Fig. 1, the second path is shown/addressed in relation to Fig. 2.

Fig. 1 shows the intracardiac device during insertion when the distal openings are distal and the proximal openings are proximal the hemostasis valve, forming the first type of leak path. This configuration may occur, for example, during insertion of the intracardiac device into the patient's vasculature as the device is passed through hemostasis valve 120. Sleeve 100 covers proximal openings 110. Sleeve 100 covers proximal openings 110 such that fluid cannot flow through distal openings 112 to proximal openings 110 via cannula 106, and leak out of the intracardiac device through proximal openings 110.

In some configurations, for example, flexible tip 116 is distal hemostasis valve 120 such that flexible tip 116 may be in fluid communication with the patient's vasculature. Distal openings 112 may be proximal hemostasis valve 120, such that they are on the other side of hemostasis valve 120 with respect to flexible tip 116. A pathway for fluid (e.g., purge fluid, blood, body fluid) to exit proximally out of hemostasis valve 120 may open at this junction, as the diameter of flexible tip 116 is smaller than the diameter of distal openings 112, creating an opening between the intracardiac device and hemostasis valve 120. Sleeve 100 covers proximal openings 110 and distal openings 112 such that fluid cannot flow through the opening created by the change in the diameter between flexible tip 116 and distal openings 110. As demonstrated in FIGS. 1-2, the intracardiac device cannot fully enter introducer 118 (i.e., a portion of the intracardiac device (e.g., the proximal end of elongate catheter 114) will always be proximal hemostasis valve 120 and introducer 118). At least one advantage of configuring the intracardiac device to include sleeve 100 is that fluids are prevented from leaking out of hemostasis valve 120 via introducer 118. At least another advantage is that the stiffness of the placement system is increased, (specifically the stiffness of cannula 106), enabling an easier entry of the intracardiac device into the patient's vasculature.

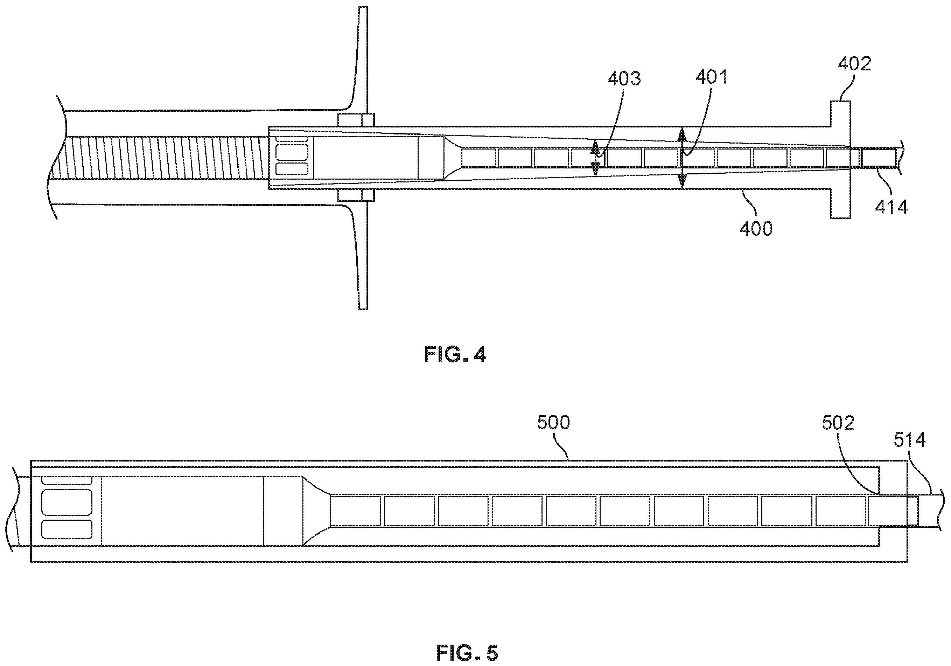

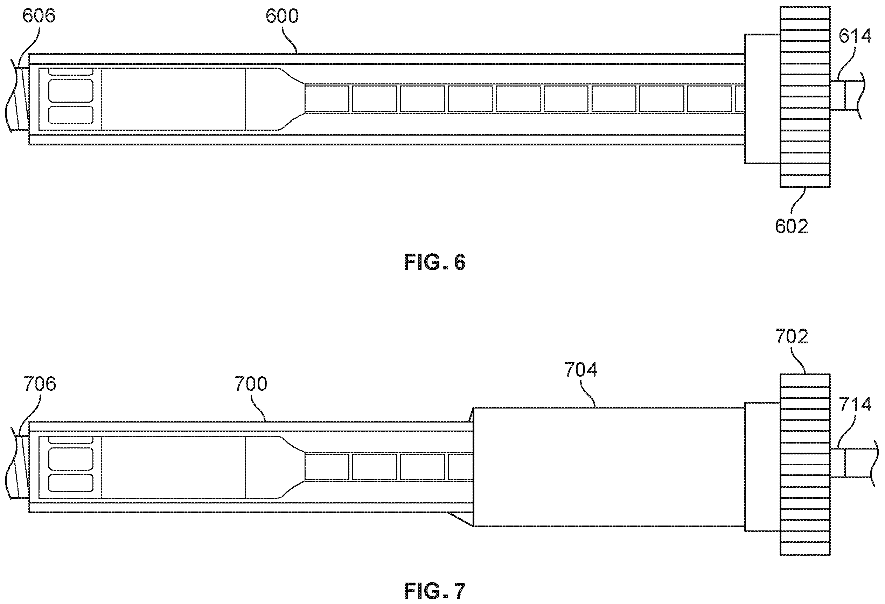

In various implementations described below, sleeve 100 is effectively blocked from passing through hemostasis valve 120. In some configurations, for example, sleeve 100 may have an outer lip (as shown in FIG. 4 and as described below) or a lock mechanism (as shown in FIG. 6 and as described below) that blocks sleeve 100 from passing through hemostasis valve 120. Various implementations of sleeves and placement systems are further described below in relation to FIGS. 2-9.

Fig. 2 shows the intracardiac device during insertion when the flexible tip is distal and the distal openings is proximal the hemostasis valve, forming the first type of leak path, according to some implementations. Sleeve 100 covers proximal openings 110. Sleeve 100 covers proximal openings 110 such that fluid cannot flow through distal openings 112 to proximal openings 110 via cannula 106, and leak out of the intracardiac device through proximal openings 110. This configuration may occur, for example, during insertion of the intracardiac device into the patient's vasculature to prevent the inserter of the intracardiac device from being sprayed with fluid during the insertion. The proximal end of sleeve 100 is tapered such that it fits snugly around catheter 114. For example, the tapered proximal end of sleeve 100 may include an O-ring to create a fluid tight connection between the proximal end of sleeve 100 and catheter 114. This configuration may ensure, for example, that liquid does not exit out of the connection point between the proximal end of catheter 110 and the proximal end of sleeve 100. In some implementations, sleeve 100 may be slidably disposed over the intracardiac device. Sleeve 100 may be made of a thermoplastic, or any other suitable material.

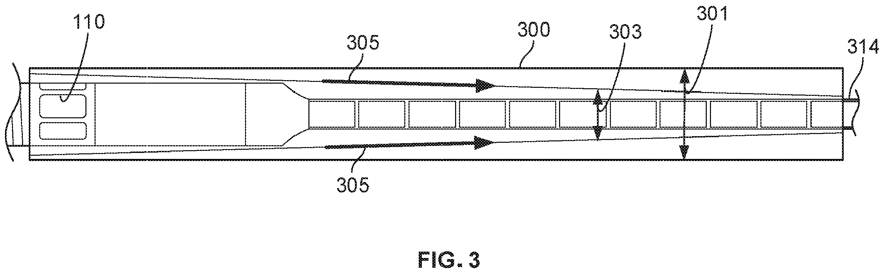

FIG. 3 shows sleeve 300 with a tapered inner diameter configured to fit snugly around elongate catheter 314, according to certain implementations. The placement system includes sleeve 300 with outer diameter 301 and inner diameter 303, elongate catheter 314, and proximal openings 110. Sleeve 300 has a lumen with a cross section. In some implementations, the lumen's cross section may taper from a wider distal end to a narrower proximal end. The lumen may taper such that the narrower proximal end fits snugly around elongate catheter 314 to prevent fluid from leaking out of the connection point between the proximal end of sleeve 300 and elongate catheter 314. As shown in FIG. 3, in some implementations, outer diameter 301 of sleeve 300 may be constant. In some implementations, outer diameter 301 of sleeve 300 may taper in the same or opposite direction of inner diameter 303 of sleeve 300. For example, in some configurations, outer diameter 301 of sleeve 300 may taper at the same angle as the taper of inner diameter 303 of sleeve 300, enabling sleeve 300 to have a constant width throughout.

In some implementations, taper 305 extends all the way through the sleeve. For example, inner diameter 303 of sleeve 300 may taper linearly from a wider distal end to a narrower proximal end. Inner diameter 303 of sleeve 300 may be larger than the diameter of distal openings 110 such that distal openings may slide distally through sleeve 300 without friction (e.g., friction caused by contact between the distal end of sleeve 300 and distal openings 110).

In some implementations, only a portion of inner diameter 303 is tapered. For example, in some configurations, inner diameter 303 may start tapering at the halfway point of the sleeve. As another example, in some configurations, inner diameter 303 could start tapering at a position such that the entire intracardiac device may fit inside the sleeve proximal hemostasis valve 120.

FIG. 4 shows sleeve 400 with lip 402 extending radially outward from an outer surface of sleeve 400 with outer diameter 401 and inner diameter 403, according to certain implementations. Lip 402 is configured on the proximal end of sleeve 400 and prevents the proximal end of sleeve 400 from passing through hemostasis valve 120. Lip 402 may be made of the same material as sleeve 400. Lip 402 may be made from a material stiff enough to prevent deformations of lip 402 (i.e., to ensure that lip 402 does not bend, snap, or cause any other deformation that would allow sleeve 400 to be fully inserted into introducer 118). The outer diameter of lip 402 may be any length that enables lip 402 to prevent sleeve 400 from being fully inserted into introducer 118.

In some configurations, for example, sleeve 400 may be similar to sleeve 300 of FIG. 3, where inner diameter 403 of sleeve 400 tapers to fit snugly around elongate catheter 414. The inner diameter of sleeve 400 may taper in the ways described above with reference to FIG. 3.

FIG. 5 shows sleeve 500 with inner lip 502, according to certain implementations. FIG. 5 shows an alternate implementation of FIGS. 3-4; instead of having a sleeve with an inner diameter that is tapered, sleeve 500 is configured such that it has two distinct diameters. Sleeve 500 may have a wide diameter through a distal portion of sleeve 500 and a narrow diameter through a proximal portion of sleeve 500. The narrow diameter may be approximately equal to the outer diameter of elongate catheter 514, creating a snug fit between sleeve 500 and elongate catheter 514. In some configurations, the implementation of FIG. 5 may be combined with that shown in FIG. 4.

As shown in FIG. 5, in some implementations, the distal outer and inner diameters of sleeve 500 may be constant, and wider than the proximal outer and inner diameters of sleeve 500. In some configurations, for example, the transition point from the wider distal diameter to the narrower proximal diameter of sleeve 500 may occur at a place on sleeve 500 that allows the entire intracardiac device to fit in the portion of the sleeve with the wider diameter.

FIG. 6 shows a placement system that includes sleeve 600 with handle 602 extending radially outward from an outer surface of sleeve 600, where an opening through locking mechanism 602 (e.g., a Tuohy Borst locking mechanism) is configured such that elongate catheter 614 slips through the opening, according to some implementations. The placement system includes sleeve 600, locking mechanism 602, cannula 606, and elongate catheter 614. Locking mechanism 602 is configured to fit snugly around elongate catheter 614 such that it minimizes fluid leaking out of hemostasis valve 120 via introducer 118. In some configurations, for example, locking mechanism 602 may be configured to fit snugly around elongate 614 by rotating locking mechanism 602 (e.g., rotating clockwise) such that locking mechanism 602 clamps down on sleeve 600 or elongate catheter 614 preventing sleeve 600 from sliding freely over the intracardiac device.

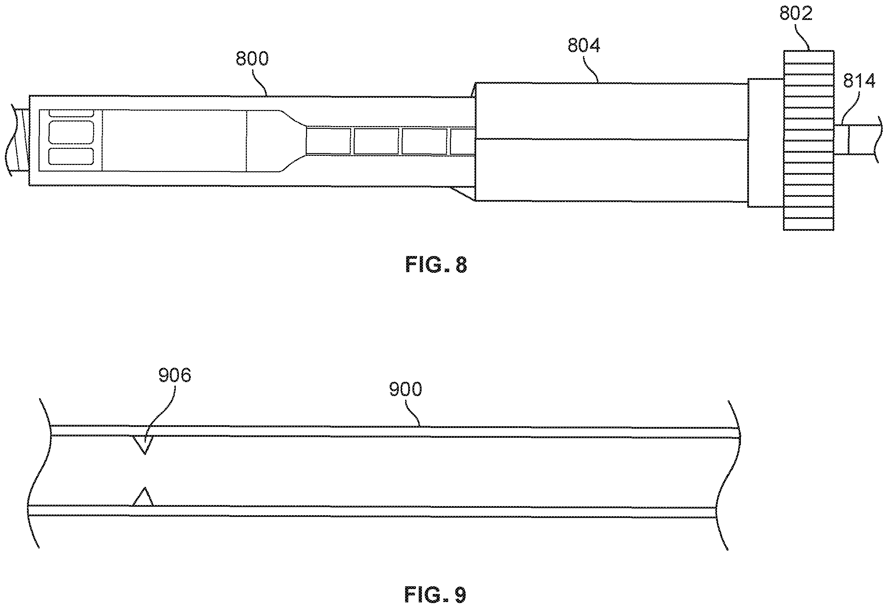

In some configurations, for example, locking mechanism 602 may be removable from sleeve 600. Locking mechanism 602 may be removed from sleeve 600 by rotating (e.g., unscrewing) locking mechanism 602 until locking mechanism has separated from sleeve 600. In some configurations, for example, locking mechanism 602 may separate into two pieces through a sliding motion, as further described in FIG. 8. In some implementations, locking mechanism 602 may further include a handle that extends distally over sleeve 600, as described in FIGS. 7-8.At least some of the advantages of configuring locking mechanism 602 to fit snugly around elongate catheter 614 include minimizing fluid that flows from distal openings 112 to proximal openings 110 and through hemostasis valve 120 to prevent leaks.

FIG. 7 shows a placement system that includes sleeve 700 with locking mechanism 702 and handle 704 extending radially outward from an outer surface of sleeve 700, where an opening through locking mechanism 702 (e.g., a Tuohy Borst locking mechanism) is configured such that elongate catheter 714 slips through the opening. The placement system includes sleeve 700, locking mechanism 702, handle 704, cannula 706, and elongate catheter 714. Handle 704 may be made of the same material as locking mechanism 702. In some implementations, for example, handle 704 may be integrally formed with the sleeve. In some implementations, handle 704 may be ergonomically sized (i.e., sized such that a human hand may fit comfortably around it (e.g., the size of a pencil grip)).

In some configurations, for example, handle 704 may be removable. For example, handle 704 may be configured to clip onto sleeve 700 (e.g., like a clamshell). In some configurations, for example, handle 704 may be configured to peel off sleeve 700 in a manner similar to introducer 118 uncoupling from hemostasis valve 120.

In some configurations, for example, handle 704 may be integrated into locking mechanism 702. Handle 704 may be made of the same material as sleeve locking mechanism 702 to form a continuous structure. Handle 704 may be made from a material stiff enough to prevent deformations of handle 704. At least one benefit of configuring the intracardiac device to include handle 704 is that the stiffness of the placement system is increased, including sleeve 700 and cannula 706, enabling an easier entry of the intracardiac device into the patient's vasculature.

FIG. 8 shows a placement system that includes sleeve 800 with locking mechanism 802 and handle 804 extending radially outward from an outer surface of sleeve 800, where an opening through locking mechanism 802 (e.g., a Tuohy Borst locking mechanism) is configured such that elongate catheter 814 slips through the opening. The placement system includes sleeve 800, locking mechanism 802, handle 804, cannula 806, and elongate catheter 814. Handle 804 may be configured as two separate pieces that are coupled together around sleeve 800 to form handle 804. In some implementations, for example, the two separate pieces may be coupled together by being configured to snap together around sleeve 800. In some implementations, for example, the two separate pieces may be coupled together around sleeve 800 via a ring that screws axially onto the two handle pieces (e.g., two halves of the handle). In some implementations, for example, the two separate pieces may be coupled together and held in place around sleeve 800 via a removable pin that locks the two halves in place. The removal of the pin allows the two separate pieces to separate and be removed from sleeve 800. In some implementations, handle 804 may be configured as two separate pieces coupled around sleeve 800 that split apart when moved distally in a sliding motion towards the proximal side of hemostasis valve 120. At least one benefit of a handle with two pieces are that the handle is able to be removed from the sleeve, allowing for more flexibility during the insertion procedure.

FIG. 9 shows sleeve 900 with inner tabs 906 extending radially inward from an inner surface of sleeve 900, according to certain implementations. Inner tabs 906 are configured inside the proximal end of sleeve 900 and form a locking mechanism, preventing the proximal end of sleeve 900 from passing through hemostasis valve 120. Inner tabs 906 may be made from a material stiff enough to prevent deformations of inner tabs 906 (i.e., to ensure that inner tabs 906 does not bend, snap, break, or cause any other deformation that would allow sleeve 900 to be fully inserted into introducer 118). Inner tabs 906 may include one or more tabs. The length of inner tabs 906 may be any length that enables inner tabs 906 to prevent sleeve 900 from being fully inserted into introducer 118 while still allowing the intracardiac device to pass through the opening between inner tabs 906.

In some implementations, inner tabs 906 include a plurality of tabs, where each tab includes a living hinge and a protrusion configured to fit within proximal openings 110. In some configurations, for example, inner tabs 906 may be configured to snap into a locked position at proximal openings 110. In some configurations, for example, inner tabs 906 may be configured to snap into a locked position at distal openings 112. And in some configurations, for example, inner tabs 906 may be configured to snap into a locked position at both proximal openings 110 and distal openings 112. Inner tabs 906 may be configured to be released from the locked position by the application of a radially inward force to a distal portion of sleeve 900.

In some implementations, each tab in inner tabs 906 may be sized and shaped to cover an opening in distal openings 112 or proximal openings 110 when in the locked position. In some configurations, for example, each opening in distal openings 112 or proximal openings 110 may be covered by a tab in the plurality of inner tabs 906. At least one advantage of including inner tabs 906 is that fluid leaks are prevented from flowing out of hemostasis valve 120 as the inner tabs, when locked into distal openings 112, proximal openings 110, or both distal openings 112 and proximal openings 110, preventing fluid from flowing out of the proximal side of hemostasis valve 120. At least one further advantage of including inner tabs 906 to lock into distal openings 112, proximal openings 110, or both distal openings 112 and proximal openings 110 is that the locked position provides a very stable and tight seal, such that fluid will not leak out of the hemostasis valve when high pressures and forces are applied during insertion.

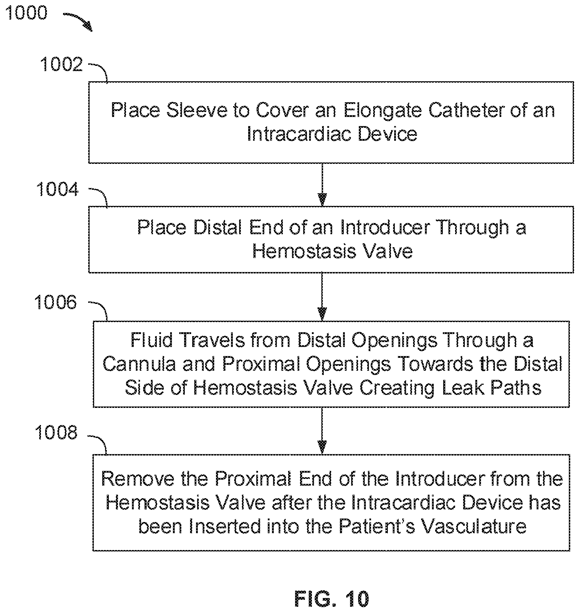

FIG. 10 shows a flowchart for preventing leaks across a hemostasis valve, according to certain implementations. Process 1000 starts at step 1002, where a sleeve is placed to cover an elongate catheter of an intracardiac device. The sleeve is configured to fit snugly around the intracardiac device, as described above in reference to FIGS. 1-9. For example, the sleeve may be tapered to create a snug connection at the proximal end of the sleeve with the elongate catheter, as described above in reference to FIGS. 3-4. As another example, the sleeve may have a locking mechanism configured to lock onto the elongate catheter at the proximal end of the catheter, as described above in reference to FIGS. 6-9. Following step 1002, at step 1004 a distal end of an introducer is placed through a hemostasis valve, entering from the proximal side of the hemostasis valve and exiting through the distal side of the hemostasis valve. The introducer is positioned through the hemostasis valve to guide the intracardiac device into the patient's vasculature. This process is illustrated in FIGS. 1-2, as described above. At step 1006, where fluid travels from distal openings through a cannula and proximal openings towards the distal side of the hemostasis valve there are potential leak paths, as described in reference to FIG. 1. For example, as discussed above in relation to FIG. 1, a potential leak pathway occurs when, during insertion, a flexible tip positioned distal the hemostasis valve and proximal openings and distal openings are both proximal the hemostasis valve. The lumen through the flexible tip and to the distal openings can create a pathway for fluid to leak through the hemostasis valve. As another example, as discussed above in relation to FIG. 2, a potential leak pathway occurs when, during insertion, the distal openings are distal the hemostasis valve and the proximal openings are proximal the hemostasis valve. Fluid flows through distal openings through the cannula and out the proximal openings, creating a pathway for fluid to leak through the hemostasis valve. The sleeve blocks said fluid from flowing out of the hemostasis valve, preventing the fluid from leaking (and spraying) out of the hemostasis valve via the introducer. At step 1008, the proximal end of the introducer is removed from the hemostasis valve after the intracardiac device has been inserted into the patient's vasculature. Though the steps of process 1000 are recited in a specific order, the steps can be completed in any order.

The foregoing is merely illustrative of the principles of the disclosure and the apparatuses can be practiced by other than the described aspects, which are presented for purposes of illustration and not of limitation. It is to be understood that the apparatuses disclosed herein, while shown for use in percutaneous insertion of blood pumps, may be applied to apparatuses in other applications requiring hemostasis.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Examples of changes, substitutions and alterations are ascertainable by one skilled in the art and could be made without departing from the scope of the information disclosed herein. All references cited herein are incorporated by reference in their entirety and made part of this application.

Preferred Embodiments of the invention are described in the following paras:
1. A intracardiac device placement system comprising:
   an intracardiac device comprising a pump and a cannula, the pump having a pump housing, a rotor, and an opening positioned proximal of the pump housing, the cannula having a proximal end that interfaces with the distal end of the pump housing and a distal end with at least one distal opening, the pump being configured to be operated by a motor;
   an elongate catheter extending proximal of the pump housing;
   a sleeve configured to be removably disposed over a portion of the pump and covering at least one of the distal openings and the proximal openings; and
   an introducer comprising a tubular section with a hemostasis valve, the introducer configured to introduce the pump and the sleeve into patient vasculature while preventing the sleeve from wholly passing through the proximal hemostasis valve.
2. The placement system of para 1, further comprising a flexible projection extending distally away from the distal end of the cannula, the distal opening forming a window positioned between the flexible projection and the distal end of the cannula, and the sleeve configured to be removably disposed over the window.
3. The placement system of para 2, wherein the sleeve covers distal and proximal openings.
4. The placement system of any of the above paras, wherein the sleeve comprises a proximal end and a distal end, and wherein an inner diameter of the proximal end of the sleeve approximates an outer diameter of the elongate catheter such that, when disposed over the pump, the sleeve is fluidly sealed at its proximal end.
5. The placement system of any of the above paras, wherein the sleeve defines a lumen with a cross sectional diameter, the cross sectional diameter tapering from the distal end to the proximal end.
6. The placement system of any of the above paras, wherein the elongate catheter is coupled on its distal end to the pump housing and wherein the pump further comprises a drive cable extending through the elongate catheter.
7. The placement system of any of the above paras, wherein a portion of the sleeve is stiffer than at least one of: the elongate catheter or the cannula.
8. The placement system of any of the above paras, wherein the sleeve is configured to prevent fluid from flowing out of one of the inlet openings or the outlet openings.
9. The placement system of any of the above paras, wherein the sleeve covers the inlet openings such that when the pump is located across the hemostasis valve, with the flexible atraumatic projection positioned distal of the hemostasis valve but the inlet openings positioned proximal of the hemostasis valve, the sleeve prevents fluid from flowing from the vasculature of the patient through the lumen of the flexible atraumatic projection and out of the outlet openings.
10. The placement system of any of the above paras, wherein the sleeve covers the outlet openings such that when the pump is located across the hemostasis valve, with the inlet openings positioned distal of the hemostasis valve but the outlet openings positioned proximal of the hemostasis valve, the sleeve prevents fluid from flowing from the vasculature of the patient through the lumen of the flexible atraumatic projection or the inlet openings, and out of the outlet openings.
11. The placement system of any of the above paras, further comprising an ergonomic handle positioned over a portion of the sleeve.
12. The placement system of para 11, wherein the handle is integrally formed with the sleeve.
13. The placement system of para 11, wherein the handle is formed of at least two pieces and is removable from the sleeve.
14. The placement system of para 13, wherein the at least two pieces of the handle are held in place around the sleeve by a removable pin.
15. The placement system of para 13, wherein the at least two pieces of the handle are held in place around the sleeve by a holding ring that screws into at least one of the at least two pieces of the handle.
16. The placement system of any of the above paras, wherein the sleeve comprises a lip extending from an inner surface of a proximal end of the sleeve, the lip defining an inner diameter that approximates an outer diameter of the elongate catheter.
17. The placement system of any of the above paras, wherein the sleeve comprises a lip extending from an outer surface of the sleeve, the lip sized and shaped to abut against the hemostasis valve.
18. The placement system of any of the above paras, further comprising a locking mechanism configured to prevent the sleeve from sliding freely over the pump or a portion of the pump.
19. The placement system of para 18, wherein the locking mechanism comprises:
   a ring configured to rotate from a first, unlocked position to a second, locked position,
   wherein in the second, locked position the ring clamps onto the sleeve, the elongate catheter, or both.
20. The placement system of para 18, wherein a handle positioned over a portion of the sleeve comprises the locking mechanism.
21. The placement system of para 18, wherein the locking mechanism comprises a plurality of tabs, each tab of the plurality of tabs comprising a living hinge and a protrusion configured to fit at least partially within the inlet openings, the outlet openings or both, such that the plurality of tabs snap into a locked position at the inlet openings, the outlet openings or both.
22. The placement system of para 21, wherein the tabs are configured to be released from the locked position by applying a radially inward force to a distal portion of the sleeve.
23. The placement system of para 21, wherein each tab of the plurality of tabs is sized and shaped to cover one of the inlet openings in the locked position, and wherein each opening of the inlet openings is covered by a tab of the plurality of tabs.
24. The placement system of para 21, wherein each tab of the plurality of tabs is sized and shaped to cover one of the outlet openings in the locked position, and wherein each opening of the outlet openings is covered by a tab of the plurality of tabs.

## Claims

1. An intracardiac device placement system comprising:
a pump (107),
a cannula (106; 606; 706; 806),
a sleeve (100; 300; 400; 500; 600; 700; 800; 900) and
an introducer (118),
wherein the sleeve (100; 300; 400; 500; 600; 700; 800; 900) is configured to be removably disposed over a portion of the pump (107) or the cannula (106; 606; 706; 806) and to cover at least one of distal openings (112) and proximal openings (110) of the pump (107) or the cannula (106; 606; 706; 806), and
wherein the introducer (118) comprises a hemostasis valve (120) and is configured to introduce the pump (107) and the sleeve (100; 300; 400; 500; 600; 700; 800; 900) into a patient's vasculature while preventing the sleeve (100; 300; 400; 500; 600; 700; 800; 900) from wholly passing through the proximal hemostasis valve (120).

2. The placement system of claim 1, wherein
the pump (107) and the cannula (106) form part of an intracardiac device (122),
the pump (107) having a pump housing (109), a rotor, and an opening (110) positioned proximal of the pump housing (109),
the cannula (106; 606; 706; 806) having a proximal end that interfaces with the distal end of the pump housing (109) and a distal end with at least one distal opening (112), and
the pump (107) being configured to be operated by a motor; and
the placement system further comprises an elongate catheter (114; 314; 414; 514; 614; 714; 814) extending proximal of the pump housing (109).

3. The placement system of claim 2, further comprising a flexible projection (116) extending distally away from the distal end of the cannula (106; 606; 706; 806), the distal opening (112) forming a window positioned between the flexible projection (116) and the distal end of the cannula (106; 606; 706; 806), and the sleeve (100; 300; 400; 500; 600; 700; 800; 900) configured to be removably disposed over the window, wherein the sleeve (100; 300; 400; 500; 600; 700; 800; 900) preferably covers distal and proximal openings (112, 110).

4. The placement system of claim 2 or 3, wherein the sleeve (100; 300; 400; 500; 600; 700; 800; 900) comprises a proximal end and a distal end, and wherein an inner diameter of the proximal end of the sleeve (100; 300; 400; 500; 600; 700; 800; 900) approximates an outer diameter of the elongate catheter (114; 314; 414; 514; 614; 714; 814) such that, when disposed over the pump (107), the sleeve (100; 300; 400; 500; 600; 700; 800; 900) is fluidly sealed at its proximal end.

5. The placement system of any one of the above claims, wherein the sleeve (300; 400) defines a lumen with a cross sectional diameter (303; 403), the cross sectional diameter (303; 403) tapering from the distal end to the proximal end.

6. The placement system of any one of claims 2 to 5, wherein the elongate catheter (114; 314; 414; 514; 614; 714; 814) is coupled on its distal end to the pump housing (109) and wherein the pump (107) further comprises a drive cable extending through the elongate catheter (114; 314; 414; 514; 614; 714; 814).

7. The placement system of any one of the above claims, wherein a portion of the sleeve (100; 300; 400; 500; 600; 700; 800; 900) is stiffer than at least one of: the elongate catheter (114; 314; 414; 514; 614; 714; 814) or the cannula (106; 606; 706; 806).

8. The placement system of any one of the above claims, wherein the sleeve (100; 300; 400; 500; 600; 700; 800; 900) is configured to prevent fluid from flowing out of one of the inlet openings or the outlet openings (112, 110).

9. The placement system of any one of claims 3 to 8, wherein the sleeve (100; 300; 400; 500; 600; 700; 800; 900) covers the inlet openings (112) such that when the pump (107) is located across the hemostasis valve (120), with the flexible atraumatic projection (116) positioned distal of the hemostasis valve (120) but the inlet openings (112) positioned proximal of the hemostasis valve (120), the sleeve (100; 300; 400; 500; 600; 700; 800; 900) prevents fluid from flowing from the vasculature of the patient through the lumen of the flexible atraumatic projection (116) and out of the outlet openings (110).

10. The placement system of any one of the above claims, wherein the sleeve (100; 300; 400; 500; 600; 700; 800; 900) covers the outlet openings (110) such that when the pump (107) is located across the hemostasis valve (120), with the inlet openings (112) positioned distal of the hemostasis valve (120) but the outlet openings (110) positioned proximal of the hemostasis valve (120), the sleeve (100; 300; 400; 500; 600; 700; 800; 900) prevents fluid from flowing from the vasculature of the patient through the lumen of the flexible atraumatic projection (116) or the inlet openings (112), and out of the outlet openings (110).

11. The placement system of any one of the above claims, further comprising an ergonomic handle (704; 804) positioned over a portion of the sleeve (700; 800), wherein the handle (704) preferably comprises one of the following features:
the handle (704) is integrally formed with the sleeve (700); and
the handle (804) is formed of at least two pieces and is removable from the sleeve (800), wherein the at least two pieces of the handle (804) are preferably held in place around the sleeve (800) either by a removable pin or by a holding ring that screws into at least one of the at least two pieces of the handle (804).

12. The placement system of any one of the above claims, wherein the sleeve (500) comprises a lip (502) extending from an inner surface of a proximal end of the sleeve (500), the lip (502) defining an inner diameter that approximates an outer diameter of the elongate catheter (514).

13. The placement system of any one of the above claims, wherein the sleeve (400) comprises a lip (402) extending from an outer surface of the sleeve (400), the lip sized and shaped to abut against the hemostasis valve (120).

14. The placement system of any one of the above claims, further comprising a locking mechanism (602; 702; 802) configured to prevent the sleeve (600; 700; 800) from sliding freely over the pump (107) or a portion of the pump (107), wherein the locking mechanism (602; 702; 802) preferably comprises one of the following features:
the locking mechanism (602; 702; 802) comprises a ring configured to rotate from a first, unlocked position to a second, locked position, wherein in the second, locked position the ring clamps onto the sleeve (600; 700; 800), the elongate catheter (614; 714; 814), or both;
the locking mechanism (702; 802) is comprised by a handle (704; 804) positioned over a portion of the sleeve (700; 800); and
the locking mechanism (602; 702; 802) comprises a plurality of tabs (906), each tab of the plurality of tabs (906) comprising a living hinge and a protrusion configured to fit at least partially within the inlet openings (112), the outlet openings (110) or both, such that the plurality of tabs (906) snap into a locked position at the inlet openings (112), the outlet openings (110) or both, wherein the plurality of tabs (906) preferably comprises one of the following features:
the tabs (906) are configured to be released from the locked position by applying a radially inward force to a distal portion of the sleeve (900);
each tab of the plurality of tabs (906) is sized and shaped to cover one of the inlet openings (112) in the locked position and each opening of the inlet openings (112) is covered by a tab of the plurality of tabs (906); and
each tab of the plurality of tabs (906) is sized and shaped to cover one of the outlet openings (110) in the locked position and each opening of the outlet openings (110) is covered by a tab of the plurality of tabs (906).

15. The placement system of any one of the above claims, wherein the introducer (118) comprises a tubular section with the hemostasis valve (120)
